# EUROPÄISCHE PATENTSCHRIFT

(11) **EP 0 575 568 B1**
(45) Veröffentlichungstag und Bekanntmachung des Hinweises auf die Patenterteilung: **27.12.1996**
(21) Anmeldenummer: 92922223.0
(22) Anmeldetag: 24.10.1992
(51) Int. Cl.: A61B 1/00, A61M 39/00

(54) **VERSCHLUSSSYSTEM FÜR EINEN INSTRUMENTENDURCHLASS**
CLOSURE SYSTEM FOR AN INSTRUMENT OUTLET
SYSTEME DE FERMETURE POUR UN PASSAGE D'INSTRUMENT

(30) Priorität: 31.10.1991 DE 4135983
(43) Veröffentlichungstag der Anmeldung: 29.12.1993
(73) Patentinhaber: LASER, Helmut, Dipl.-Tech., D-10405 Berlin (DE)
(72) Erfinder: LASER, Helmut, D-10405 Berlin (DE); HEIN, Peter, W-1615 Zeuthen (DE)
(74) Vertreter: Bock, Gerhard
(86) Internationale Anmeldenummer: EP9202439
(87) Internationale Veröffentlichungsnummer: WO9308728

(56) Entgegenhaltungen:
- EP-A- 0 198 407
- DE-A- 2 800 607
- DE-A- 3 242 870
- DE-U- 7 430 345

## Beschreibung

Die Erfindung betrifft ein Verschlußsystem für einen Instrumentendurchlaß, insbesondere für laparoskopische Einstichgeräte und zum Verschließen von Instrumentier- und Spülkanälen.

Es sind verschiedene, nach unterschiedlichen Prinzipien aufgebaute Verschlußsysteme für Instrumentendurchlässe bekannt. Diesen bekannten Verschlußsystemen haften folgende Nachteile an:
Das als Trompetenventil bekannte Verschlußsystem muß beim Einführen eines Instrumentes von Hand betätigt und zu Reinigungszwecken in seine Bestandteile zerlegt werden. Die Fertigung des Trompetenventils ist aufwendig und erfordert eine hohe Präzision der Einzelteile. Bei dem als automatisches Klappenventil bekannten Verschlußsystem ist eine Verschlußklappe auf einer dünnen Achse befestigt und wird mittels einer Feder in die verschlossene Lage gebracht. Die so befestigte Klappe ist einer Reinigung schwer zugänglich. Eine Beschädigung der Frontlinse eines Endoskops, ist bei schnellem Einführen nicht auszuschließen.
In der Erfindungsbeschreibung DE-GM 74 30 345 ist ein Verschlußsystem beschrieben, das eine an einem Gelenk befestigte Klappe enthält, die durch ein Dauermagnetsystem in die verschlossene Lage gebracht wird. Eine Reinigung ist hier ebenfalls schwierig.
In DE 32 42 870 A1 ist ein Verschlußsystem beschrieben, bei dem eine ggf. einseitig angelenkte Verschlußklappe eingesetzt ist, die einen Ringmagneten enthält, der mit einem Ringmagneten des Klappensitzes korrespondiert. Dabei kann die Klappe zum Schutz einzuführender Instrumente zusätzlich mit einem Kunststoff überzogen sein. Bei dieser Lösung ist es von Nachteil, daß, wenn die Klappe nicht angelenkt ist, diese beim Einführen ferromagnetischer Instrumente von diesen leicht mitgenommen werden kann, was zum einen eine Behinderung für den Operateur darstellt und zum anderen ein absolut sicheres Verschließen der Klappe nach der Instrumentenentnahme nicht gewährleistet. Desweiteren ist in DE 28 00 607 A1 ein Operations-Laparoskop beschrieben, bei dem der Verschluß über eine ferromagnetische Kugel erfolgt, die durch einen ringförmigen Magneten im Ventilsitz angezogen wird. Die ferromagnetische Kugel wird dabei in einem trichterförmigen Ventilsitz geführt. Werden bei diesem Vorschlag Instrumente mit planen Frontflächen eingeführt, kann es ebenfalls sehr leicht zu einem vollständigen Abheben der Kugel kommen, so daß auch hier ein sicheres Verschließen nach der Instrumentenentnahme nicht gewährleistet ist. Um dem zu begegnen, werden bei diesem Vorschlag an der Frontfläche speziell ausgebildete Instrumente benötigt, die ein seitliches Verdrängen der Kugel im trichterförmigen Sitz bewirken und einen Schutz bspw. von Endoskopoptiken bedingen. Diese Maßnahme stellt einen erheblichen gerätetechnischen Mehraufwand dar. Optiken gebräuchlicher Endoskope können durch diesen Vorschlag in gleicher Weise, wie bei sonstigen Kugelventilen beschädigt werden. Ein Verschlußsystem nach DE 28 00 607 A1 läßt sich zwar gut reinigen, beansprucht aber eine große Bauform; durch die Masse der Kugel wird eine hohe Magnetkraft benötigt und weshalb das System besonders schwer ist.

Der Erfindung liegt die Aufgabe zugrunde, ein gattungsgemäßes Verschlußsystem für Instrumentendurchlässe zu schaffen, das neben einer hohen Verschlußsicherheit eine problemlose Reinigung und einen einfachen sicheren Zusammenbau gewährleistet. Weiterhin ist es Aufgabe der Erfindung, das Verschlußsystem selbst so auszubilden, daß eine Beschädigung der Frontlinsen eines Endoskops beim Einführen in das Verschlußsystem ausgeschlossen ist, ohne daß besondere zusätzliche Maßnahmen an den Instrumenten selbst erforderlich sind.

Erfindungsgemäß wird die Aufgabe durch den Gegenstand des Anspruchs 1 gelöst.

Das erfindungsgemäße Verschlußsystem besteht aus einer um eine außerhalb des Instrumentendurchlasses liegende Achse schwenkbaren Verschlußklappe, die durch eine in Schließrichtung wirkende Magnetkraft wenigstens eines Dauermagneten beaufschlagt ist, wobei die Verschlußklappe als schwimmend gelagerte ferromagnetische Scheibe ausgebildet ist, die in einem mit Abschrägungen versehenen Sitz umfangsseitig zentriert ist und die Anordnung des oder der Dauermagneten, derart erfolgt, daß auf die Verschlußklappe in einem definierten Umfangsabschnitt eine stärkere Magnetkraft einwirkt als an den übrigen Umfangsabschnitten.
Der Dauermagnet umgibt den Instrumentendurchlaß der Einrichtung vorteilhafterweise kreisringsegmentförmig und ist mit einem ringförmigen Flußleitstück verbunden, das mit seinem axialen kreisringsegmentförmigen Ansatz den zweiten Magnetpol bildet. Wird auf die Verschlußklappe eine Kraft in Öffnungsrichtung ausgeübt, dreht sie sich um eine Achse, die bei dem stärkeren, durch den Dauermagneten gebildeten Magnetpol liegt. Diese unsymmetrische, auf die Verschlußklappe wirkende Magnetkraft, kann auch durch mehrere, in der Umfangrichtung angeordnete Dauermagnete erreicht werden; erfindungswesentlich ist, daß ein unsymmetrisch starkes Magnetfeld entsteht. Dadurch wird erreicht, daß die ferromagnetische Verschlußklappe stets um eine Achse auf der Seite des Dauermagneten beim Ein- und Ausbringen von Instrumenten definiert geschwenkt wird. Dadurch, daß die Verschlußklappe selbst keinen Magneten enthält, ist es ausgeschlossen, daß sie beim Einbringen ferromagnetischer Instrumente mitgenommen wird. Durch die unsymmetrische Magnetkraftverteilung wird weiterhin ein vollständiges Abheben der Verschlußklappe vom Sitz beim Einbringen von Instrumenten mit planer Frontfläche wirkungsvoll vermieden.
Von Vorteil ist es, den Radius der Verschlußklappe nicht wesentlich größer auszubilden als den Abstand des Dauermagneten von der Achse des Instrumentendurchlasses, weil dadurch ein sicheres Klappen in Schließrichtung aus der geöffneten Stellung erreicht wird.

Die rotationssymmetrische Ausführung der Kammer des Verschlußsystems, die entgegen der Magnetkraft einfach aus der Kammer zu entnehmende Verschlußklappe sowie das Fehlen jeglicher Gelenk- und Federelemente ermöglicht eine einfache und problemlose Reinigung des Einstichgerätes und der Elemente des Verschlußsystems.

Es liegt im Rahmen der Erfindung, die Verschlußklappe nicht ausschließlich kreisrund auszubilden. Beispielsweise wäre auch eine angefaste Kreisscheibe bis hin zu einer rechteckigen, insbesondere quadratischen Ausbildung denkbar. Bei derartigen Ausführungen ist jedoch sicherzustellen, daß eine der geraden Berandungsabschnitte auf der Seite des Dauermagneten zu liegen kommt.

Die Erfindung wird im folgenden anhand der in den Figuren schematisch dargestellten Ausführungsbeispielen näher erläutert. Es zeigen:
- Fig. 1: ein laparoskopisches Einstichgerät mit einem Verschluß gemäß der Erfindung,
- Fig. 2: einen Schnitt entlang der Linie A-A und
- Fig. 3: zwei zur Reduzierung des Instrumentendurchlasses ineinandergefügte Einstichgeräte unterschiedlichen Durchmessers.

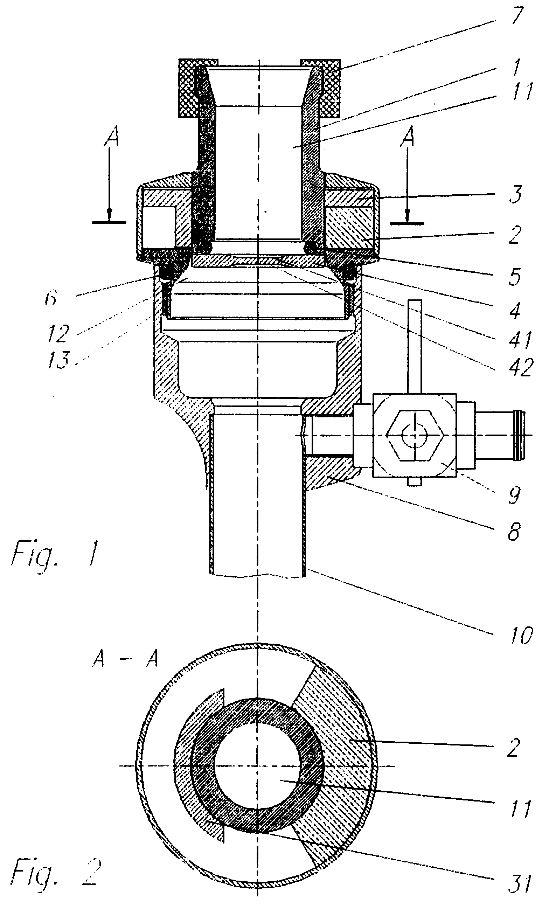

Gemäß Fig. 2 umschließt der Dauermagnet 2 den Instrumentendurchlaß 11 kreisringsegmentförmig. Mit dem Dauermagnet 2 ist das Flußleitstück 3 verbunden, dessen kreisringsegmentförmiger axialer Ansatz 31 dem Dauermagneten 2 diametral gegenüberliegt. Der Dauermagnet 2 und der Ansatz 31 bilden zwei Magnetpole in einem Magnetkreis, der durch die Verschlußklappe 4 geschlossen wird.
Durch die spezielle Anordnung des Dauermagneten 2 und des Flußleitstücks 3 ist sichergestellt, daß auf die Verschlußklappe 4 ein unsymmetrisches Magnetfeld wirkt. Durch die dort stärkere Feldkraft ist die Drehachse der Verschlußklappe 4 im Bereich des Dauermagneten 2 definiert, während die Verschlußklappe 4 beim Einführen eines Instrumentes auf der gegenüberliegenden Seite abgehoben wird. Die spezielle Anordnung des Magnetsystems bewirkt darüber hinaus, daß beim Zurückziehen eines Instrumentes die Verschlußklappe 4 sicher in den sie umfangsseitig umgebenden trichterförmigen Ventilsitz 12 zurückkehrt. Dadurch, daß der magnetische Rückschluß durch die Verschlußklappe 4 über ihre äußersten Umfangsbereiche erfolgt, das heißt, daß im Falle des Einsatzes einer rondenförmigen Verschlußklappe ihr Durchmesser nicht wesentlich größer als der Durchmesser des Instrumentendurchlasses sein soll, wird bewirkt, daß die Verschlußklappe keine andere stabile Lage als der in Verschlußstellung einnehmen kann.
Die Vertiefungen 41 und 42 auf beiden Seiten der Verschlußklappe 4 sorgen dafür, daß die Frontlinsen von Endoskopobjektiven beim Öffnen der Verschlußklappe 4 nicht beschädigt werden. Dabei sind der Verschlußklappe 4 vorteilhaft beidseitig Vertiefungen 41 und 42 gegeben, um einen lageunabhängigen Einbau zu ermöglichen.
Das laparoskopische Einstichgerät gemäß Fig. 1 besteht aus dem Ventilgehäuse 1 mit der Verschlußklappe 4 und dem mit einem Hahn 9 versehenen Kammergehäuse 8, das in das Rohr 10 übergeht. Kammergehäuse 8 und Ventilgehäuse 1 sind über das Gewinde 13 und den Dichtring 6 gasdicht miteinander verbunden.
Das Ventilgehäuse 1 ist am Eingang des Instrumentendurchlasses 11 mit einer elastischen Dichtkappe versehen. Im Inneren des Ventilgehäuses 1 befindet sich ein Magnetsystem, bestehend aus dem Dauermagneten 2 und dem Flußleitstück 3 auf dem ein kreisringsegmentförmiger, axialer Ansatz 31 vorgesehen ist. Am Ausgang des Instrumentendurchlasses 11 weist das Ventilgehäuse einen trichterförmigen Ventilsitz 12 auf, der mit einem Dichtring 5 versehen ist.
Die Ventilklappe 4 wird durch die Magnetkraft gegen den Dichtring 5 gezogen und dichtet so den Körperinnenraum des Patienten gegen die Umgebung ab. Durch den trichterförmigen Ventilsitz 12 ist ihre Position festgelegt. Dieser Ventilsitz 12 ist der äußeren Geometrie der Verschlußklappe 4 angepaßt.
Zum Reinigen und Desinfizieren des Einstichgerätes wird das Ventilgehäuse 1 aus dem Kammergehäuse 8 ausgeschraubt und die frei bewegliche Verschlußklappe 4 gegen die Magnetkraft einfach entnommen.

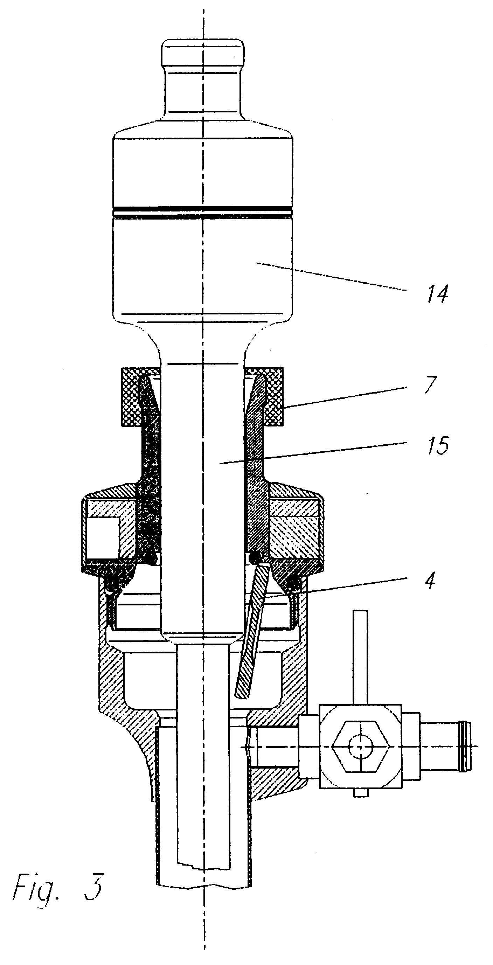

Häufig ist es während einer Operation erforderlich, den freien Durchmesser eines Einstichgerätes zur Einführung eines dünneren Instrumentes zu reduzieren. Die dazu häufig verwendeten Reduzierhülsen öffnen im allgemeinen das Ventil des Einstichgerätes, so daß es beim Instrumentenwechsel zum Teil zu erheblichen Gasverlusten kommt.
Nach einer vorteilhaften Ausführungsform, die in Fig. 3 dargestellt ist, wurde ein Einstichgerät 14 für dünnere Instrumente so gestaltet, daß es zusätzlich einen zylindrischen Schaft 15 aufweist, der in den Instrumentendurchlaß des größeren Einstichgerätes paßt.
Die Dichtkappe 7 dichtet zwischen dem Instrumentendurchlaß des größeren Einstichgerätes und dem zylindrischen Schaft 15.
Da das Einstichgerät 14 ebenfalls ein Ventil, wie oben beschrieben besitzt, kann der Instrumentenwechsel ohne größere Gasverluste durchgeführt werden. Da das Einstichgerät 14 neben der Reduzierung als vollwertiges Einstichgerät eingesetzt werden kann, reduziert sich die Anzahl der zu einem Eingriff notwendigen Geräte zugunsten universeller Instrumente.

## Patentansprüche

1. Verschlußsystem für einen Instrumentendurchlaß mit einer um eine außerhalb des Instrumentendurchlasses liegende Achse schwenkbaren Verschlußklappe (4), die durch eine in Schließrichtung wirkende Magnetkraft wenigstens eines Dauermagneten (2) beaufschlagt ist, dadurch gekennzeichnet, daß die Verschlußklappe (4) als schwimmend gelagerte ferromagnetische Scheibe ausgebildet ist, die in einem mit Abschrägungen versehenen Sitz (12) umfangsseitig zentriert ist und die Anordnung des oder der Dauermagneten (2), derart erfolgt, daß auf die Verschlußklappe (4) in einem definierten Umfangsabschnitt eine stärkere Magnetkraft einwirkt als an den übrigen Umfangsabschnitten.

2. Verschlußsystem nach Anspruch 1., dadurch gekennzeichnet, daß der Dauermagnet (2) eine kreisringsegmentförmige Grundfläche aufweist, die konzentrisch am Instrumentendurchlaß anliegt.

3. Verschlußsystem nach Anspruch 1. und 2., dadurch gekennzeichnet, daß der Dauermagnet (2) mit einem den Instrumentendurchlaß umgebenden, im wesentlichen kreisringförmigen ferromagnetischen Flußleitstück (3) verbunden ist.

4. Verschlußsystem nach Anspruch 1. bis 3., dadurch gekennzeichnet, daß dem Ort der stärksten Magnetkraft des Dauermagneten diametral gegenüberliegend, außerhalb des Instrumentendurchlasses ein ferro-magnetischer axialer Ansatz (31) vorgesehen ist.

5. Verschlußsystem nach Anspruch 1., dadurch gekennzeichnet, daß die Verschlußklappe (4) als Scheibe ausgebildet ist, die mittig wenigstens einseitig mit einer Vertiefung (41, 42) versehen ist, deren Mittelpunkt auf der Mittenachse des Instrumentendurchlasses liegt.

6. Verschlußsystem nach Anspruch 1. und 5., dadurch gekennzeichnet, daß die Verschlußklappe (4) als kreisförmige Scheibe ausgebildet ist.

7. Verschlußsystem nach einem der vorstehenden Ansprüche, dadurch gekennzeichnet, daß der Dauermagnet (2) und der axiale Ansatz (31) im Verhältnis zur Verschlußklappe (4) so angeordnet sind, daß der magnetische Rückschluß über die äußersten Umfangsbereiche der Verschlußklappe (4) erfolgt.

8. Laparoskopisches Einstichgerät (14), dadurch gekennzeichnet, daß es ein Verschlußsystem nach einem der Ansprüche 1. bis 7. enthält.

9. Laparoskopisches Einstichgerät nach Anspruch 8., dadurch gekennzeichnet, daß ein Kammergehäuse mit einem Schaft (15) versehen ist, dessen Außendurchmesser bzw. Außenprofil mit dem eingangsseitigen Innendurchmesser bzw. Innenprofil eines weiteren größeren laparoskopischen Einstichgerätes korrespondiert und mit diesem dichtend verbindbar ist.

10. Laparoskopisches Einstichgerät nach Anspruch 9., dadurch gekennzeichnet, daß der Schaft (15) zylindrisch ausgebildet ist.

11. Laparoskopisches Einstichgerät nach Anspruch 9. und 10., dadurch gekennzeichnet, daß auf dem Schaft (15) umfangsseitig eine zusätzliche Dichtfläche aufgebracht ist.

## Claims

1. Closing system for an instrument passage including a closing member pivotal about an axis remote from the axis of the instrument passage, said closing member being subject to a magnetic force effective in closing direction,
characterized in that the closing member (4) is a floatingly seated ferromagnetic disc, centered via its rim portions relative to a conical seat (12), and in that at least one magnet (2), preferably a permanent magnet, is arranged in a manner that a comparatively stronger magnetic force acts upon a defined circumferential section of said closing member (4) than on the remaining circumferential portions of said closing member (4).

2. Closing system as claimed in claim 1, characterized in that said permanent magnet (2) is provided with an annular segment base face, concentrically attached to said instrument passage.

3. Closing system as claimed in claims 1 and 2, characterized in that said permanent magnet (2) is connected to a substantially ferromagnetic flux directing member (3) enclosing said instrument passage.

4. Closing system as claimed in claims 1 to 3, characterized in that a ferromagnetic axially projecting portion (31) in diametral opposition to the area of the strongest magnetic force of said permanent magnet is provided remote from said instrument passage.

5. Closing system as claimed in claim 1, characterized in that said closing member (4) is embodied as a disc centrally provided with a dip (41, 42) at least on one of its surfaces coincident with the central axis of said instrument passage.

6. Closing system as claimed in claims 1 and 5, wherein the closing member (4) is a circular disc.

7. Closing system as claimed in any of the preceding claims, wherein said permanent magnet (2) and said axially projecting portion (31) are arranged relative to said closing member (4) in a manner that the magnetic feedback takes place via the very rim portions of said closing member (4).

8. Laparoscopic puncture device, characterized in that a closing system is provided according to any of the preceding claims 1 to 7.

9. Laparoscopic puncture device as claimed in claim 8, characterized in that a chamber housing is provided with a shaft (15) the external diameter and the external profile, respectively, of which correspond to the entry diameter and internal profile, respectively, of a further and larger puncture device and being tightly connectable to the latter.

10. Laparoscopic puncture device as claimed in claim 9, characterized in that said shaft (15) is cylindrically shaped.

11. Laparoscopic puncture device as claimed in claim 9 and 10, characterized in that said shaft (15) is circumferentially provided with an additional sealing layer.

## Revendications

1. Système de fermeture pour un passage d'instruments avec un clapet (4) pivotant autour d'un axe situé en dehors du passage d'instruments; le clapet est alimenté par une force magnétique d'au moins un aimant permanent (2) agissant dans le sens de la fermeture; l'invention est caractérisée en ce que le clapet (4) est formé par une plaque ferromagnétique logée de façon flottante; la plaque est centrée, du côté du périmètre, dans un siège (12) muni de biseautages; la disposition de l'aimant permanent ou des aimants permanents (2) s'effectue de façon telle qu'une force magnétique plus importante agit sur le clapet (4) dans un segment défini du périmètre que sur les autres segments du périmètre.

2. Système de fermeture selon la revendication 1, caractérisé en ce que l'aimant permanent (2) présente une surface de base en forme de segment d'un anneau de cercle, surface adjacente, de manière concentrique, au passage d'instruments.

3. Système de fermeture selon les revendications 1 et 2, caractérisé en ce que l'aimant permanent (2) est lié à une pièce directrice (3) ferromagnétique et essentiellement en forme de segment d'un anneau de cercle, pièce qui entoure le passage d'instruments.

4. Système de fermeture selon les revendications 1 à 3, caractérisé en ce qu'il est prévu une pièce ajoutée (31) axiale et ferromagnétique en dehors du passage d'instruments, pièce qui se trouve diamétralement opposée au lieu présentant la force magnétique la plus importante de l'aimant permanent.

5. Système de fermeture selon la revendication 1, caractérisé en ce que le clapet (4) est formé par une plaque qui, du point de vue centré, est munie au moins unilatéralement d'un creux (41, 42) dont le centre se trouve sur l'axe centrale du passage d'instruments.

6. Système de fermeture selon les revendications 1 et 5, caractérisé en ce que le clapet (4) est formé par une plaque circulaire.

7. Système de fermeture selon une des revendications précitées, caractérisé en ce que l'aimant permanent (2) et la pièce ajoutée axiale (31) sont disposés de telle façon par rapport au clapet (4) que le retour magnétique s'effectue via les zones les plus éloignées du périmètre du clapet (4).

8. Appareil de piqûre laparoscopique (14), caractérisé en ce qu'il contient un système de fermeture selon une des revendications 1 à 7.

9. Appareil de piqûre laparoscopique selon la revendication 8, caractérisé en ce qu'un boîtier-chambre est muni d'une tige (15) dont le diamètre extérieur ou bien le profil extérieur correspond au diamètre intérieur ou profil intérieur, du côté de l'entrée, d'un autre appareil de piqûre laparoscopique plus grand, et caractérisé en ce que ce profil ou diamètre extérieur peut être lié au diamètre ou profil intérieur, ce qui mène à une étanchéité.

10. Appareil de piqûre laparoscopique selon la revendication 9, caractérisé en ce que la tige (15) a une forme cylindrique.

11. Appareil de piqûre laparoscopique selon les revendications 9 et 10, caractérisé en ce que, sur la tige (15), est placée une face d'étanchéité supplémentaire du côté du périmètre.
